# EUROPÄISCHE PATENTSCHRIFT

(11) **EP 1 694 630 B1**
(45) Veröffentlichungstag und Bekanntmachung des Hinweises auf die Patenterteilung: **07.05.2008**
(21) Anmeldenummer: 04820806.0
(22) Anmeldetag: 25.11.2004
(51) Int. Cl.: C07C 209/62, C07C 227/32, C07C 213/08, C07C 229/02

(54) **VERFAHREN ZUR HERSTELLUNG VON CHIRALEN ODER ENANTIOMERENANGEREICHERTEN BETA-AMINOSAEUREN, -ALDEHYDEN, - KETONEN UND GAMMA-AMINOALKOHOLEN**
METHOD FOR PRODUCING CHIRAL OR ENANTIOMER-ENRICHED BETA-AMINO ACIDS, -ALDEHYDES, -KETONES AND GAMMA-AMINO ALCOHOLS
PROCEDE DE PRODUCTION DE BETA AMINOACIDES, DE BETA ALDEHYDES, DE BETA CETONES ET DE GAMMA AMINOALCOOLS, CHIRAUX OU ENRICHIS EN ENANTIOMERES

(30) Priorität: 19.12.2003 AT 20512003; 28.05.2004 AT 9292004
(43) Veröffentlichungstag der Anmeldung: 30.08.2006
(73) Patentinhaber: DSM Fine Chemicals Austria Nfg GmbH & Co KG, 4021 Linz (AT)
(72) Erfinder: WALTHER, Jary, A-4853 Steinbach Am Attersee (AT); DE LANGE, Ben, NL-6151 GE Munstergeleen (NL); BROXTERMAN, Quirinus, Bernardus, NL-6151 JA Munstergeleen (NL); PÖCHLAUER, Peter, A-4040 Linz (AT); VAN DER SLUIS, Marcelles, NL-9712 CL Groningen (NL); UITERWEERD, Patrick, 9737 PS Groningen (NL); FALK, Heinz, A-4040 Linz (AT); ZUCKERSTÄTTER, Gerhard, A-5412 Puch bei Salzburg (AT)
(74) Vertreter: Habets, Winand
(86) Internationale Anmeldenummer: PCT/EP2004/013354
(87) Internationale Veröffentlichungsnummer: WO 2005/063682

(56) Entgegenhaltungen:
- DE-A1- 2 200 788
- US-A1- 2003 097 005
- J.A.MARSHALL ET AL: "The Direct Conversion of Olefins into Esters Through Ozonolysis" SYNLETT, 1992, Seiten 643-645, XP001206163 in der Anmeldung erwähnt
- J.A.MARSHALL ET AL: "Oxidative Cleavage of Mono-, Di-, and Trisubstituted Olefins to Methyl Esters through Ozonolysis in Methanolic NaOH" J.ORG.CHEM., Bd. 58, 1993, Seiten 3675-3680, XP009046888 in der Anmeldung erwähnt
- PATENT ABSTRACTS OF JAPAN Bd. 1996, Nr. 05, 31. Mai 1996 (1996-05-31) & JP 08 012629 A (KANEGAFUCHI CHEM IND CO LTD), 16. Januar 1996 (1996-01-16)

## Beschreibung

Die Erfindung betrifft ein Verfahren zur Herstellung von chiralen oder enantiomerenangereicherten beta-Aminosäuren, -aldehyden, -ketonen und gamma-Aminoalkoholen ausgehend von gegebenenfalls N-geschützten Homoallylaminen Chirale oder enantiomerenangereicherte beta-Aminosäuren, -aldehyde, -ketone und gamma-Aminoalkohole finden beispielsweise als chirales Hilfsmittel, chirale Liganden, Precursor für beta-Lactame, beta-Peptide oder als Ausgangsmaterial für die Herstellung von verschiedensten natürlich vorkommenden biologisch aktiven Substanzen, als chirale Synthesebausteine, als Intermediate in der Herstellung von Pharmazeutika Verwendung.

Herstellverfahren für chirale oder enantiomerenangereicherte beta-Aminosäuren, -aldehyden, -ketonen und gamma-Aminoalkohole sind bereits eine Vielzahl aus der Literatur bekannt. Die Umsetzung von Olefinen in methanolischer Natronlauge führt zu den korrespondierenden Carbonsäureestern. Diese Methoden können auch zu Herstellung von beta-Aminosäureestern verwendet werden und sind in der Literatur beschrieben: J.A. Marshall, A.W. Garofalo, R.C. Sedrani, Synlettt, 1992, 643- 645. b) *Oxidative cleavage of mono-, di-, and trisubstituted olefins to methyl esters through ozonolysis in methanolic NaOH,* J.A. Marshall, A.W. Garafalo, J.Org. Chem. 1993, 58, 3675-3680. Der Nachteil dieser Methoden ist jedoch, das Ozon im alkalischen Milieu zum größten Teil zerstört wird und nur ein geringer Teil des Ozons für die Spaltung der Doppelbindung zur Verfügung steht. Die daraus resultierende längere Reaktionszeit macht diese Verfahren äußerst unwirtschaftlich.
Ein weiterer Nachteil dieser Methode ist, dass die Aminosäureester erhalten werden. Um die freien Aminosäuren zu erhalten, muss die Esterfunktionalität in einem weiteren Verfahrenschritt verseift werden.

Gemäß WO 01/42173 werden zuerst ein Phenylglycinamid mit u.a. einem Aldehyd, wie etwa lsobutyraldehyd umgesetzt und anschließend in die korrespondierende Schiff-Base überführt. Diese Schiff-Base wird sodann durch Reaktion mit einer allylischen organometallischen Verbindung zu der entsprechenden Allylverbindung reagiert, die durch oxidative Methoden, wie etwa durch Ozonolyse, anschließender oxidativer Behandlung und abschließender Hydrogenolyse in die gewünschte beta-Aminosäure überführt wird. Die beta-Aminosäuren werden dabei nach Reinigung mittels Säulenchromatographie in einer Menge von etwa 30% erhalten. Um zum entsprechenden beta-Aminoalkohol zu gelangen, erfolgt gemäß WO 01/42173 wiederum zuerst die Ozonolyse der Allylverbindung, mit anschließender reduktiver Aufarbeitung, beispielsweise mittels NaBH₄, sowie Schutzgruppenabspaltung durch Hydrogenolyse. Dabei wird der entsprechende Aminoalkohol nach Reinigung mittels Säulenchromatographie in einer Ausbeute von etwa 47% erhalten.

Aufgabe der vorliegenden Erfindung war es, ein alternatives Verfahren zu finden, das von einfacher zugänglichen Allyledukten ausgeht und das zu den gewünschten beta-Aminosäuren, -aldehyden, -ketonen oder gamma-Aminoalkoholen in Ausbeuten von 58% bis zu 99% führt.

Gegenstand der Erfindung ist demnach ein Verfahren zur Herstellung von chiralen oder enantiomerenangereicherten beta-Aminosäuren, -aldehyden, - ketonen oder gamma-Aminoalkoholen, das dadurch gekennzeichnet ist, dass ein Allylamin der Formel 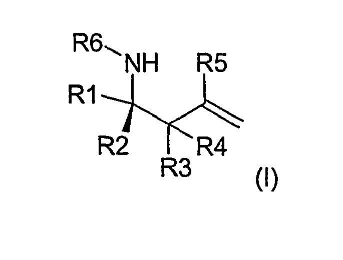 in der R1 einen Alkylrest, einen Cycloalkylrest, einen Arylrest, einen Heterocyclusrest oder ein kondensiertes oder überbrücktes Ringsystem bedeutet,
R2, R3, R4 und R5 unabhängig voneinander H oder einen Alkylrest, einen Cycloalkylrest, einen Arylrest, einen Heterocyclusrest oder ein kondensiertes oder über brücktes Ringsystem bedeuten können,
oder die Reste R1, R2, R3 und R4 untereinander Ringsysteme bilden können, die gegebenenfalls ein oder mehrere Heteroatome enthalten können,
wobei die Reste R1, R2, R3, R4 und R5 gegebenenfalls ein- oder mehrfach durch Alkyl, Phenyl, Halogen, Carbonsäurealkylester, O-geschützte Hydroxy- und Hydroxyalkylgruppen substituiert sein können und
R6 H oder eine N-Schutzgruppe ist,
a) durch Ozonolyse in einem Lösungsmittel aus der Gruppe der C₁-C₆-Carbonsäure, Wasser/Schwefelsäuregemisch, C₁-C₄-Alkohol, Ethylacetat oder Butylacetat oder Gemischen davon bei einer Reaktionstemperatur, in Abhängigkeit vom gewählten Lösungsmittel, von -40°C bis +30°C und
b) anschließender Zersetzung der peroxidhaltigen Lösung mittels eines Oxidationsmittel oder reduktiver Aufarbeitung
   in die entsprechende Aminoverbindung der Formel 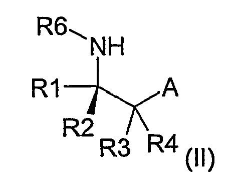 in der R1, R2, R3, R4 und R6 wie oben definiert sind,
   und A in Abhängigkeit von der Aufarbeitung für einen Rest der Formel -COOH, -C(OH)R5 oder -C(O)R5 steht, wobei R5 wie oben definiert ist, überführt wird.

Bei dem erfindungsgemäßen Verfahren werden beta-Aminosäuren, -aldehyde oder - ketone oder gamma-Aminoalkohole ausgehend von Allylverbindungen der Formel (I) hergestellt.

In der Formel (I) bedeutet R1 einen Alkylrest, einen Cycloalkylrest, einen Arylrest, einen Heterocyclusrest oder ein kondensiertes oder überbrücktes Ringsystem,
R2, R3, R4 und R5 bedeuten unabhängig voneinander H oder einen Alkylrest, einen Cycloalkylrest, einen Arylrest, einen Heterocyclusrest oder ein kondensiertes oder überbrücktes Ringsystem.
Die Reste R1, R2, R3 und R4 können gegebenenfalls auch untereinander Ringsysteme bilden, die gegebenenfalls ein oder mehrere Heteroatome enthalten können.
Somit kann beispielsweise R1 mit R2 oder mit R3 oder mit R4, oder R2 mit R3 oder R4 oder R3 mit R4 ein Ringsystem bilden. Weiters können diese Ringsysteme ein oder mehrere Heteroatome aus der Gruppe O, N oder S enthalten.

Unter Alkylrest, beispielsweise unter C₁-C₂₀-Alkylresten, sind dabei lineare oder verzweigte Alkylreste, wie etwa Methyl, Ethyl, n-Propyl, i-Propyl, n-Butyl, sek.-Butyl, tert.-Butyl, Hexyl, zu verstehen.
Bevorzugt sind dabei C₂-C₆-Alkylreste.
Cycloalkylreste sind cyclische Alkylreste, beispielsweise Cycloalkylreste mit 3-12 C-Atomen, wie etwa Cyclopropyl, Cyclohexyl, Cyclooctyl, u.s.w. Bevorzugt sind C₃-C₆-Cycloalkylreste.
Geeignete Arylreste sind aromatische Ringe und Ringsysteme mit beispielsweise 5 bis 20 C-Atomen, wie etwa Phenyl, Naphthyl, Indenyl, Fluorenyl.
Bevorzugte Arylreste sind C₆-C₁₀-Arylreste.
Unter Heterocyclusresten sind cyclische Reste mit beispielsweise 4 bis 20 C-Atomen zu verstehen, die zumindest ein Heteroatom aus der Gruppe O, S oder N enthalten und aromatische oder gesättigte oder ungesättigte aliphatische Ringe sein können, wie etwa Pyrryl, Furanyl, Thienyl, Pyridyl, Pyrimidinyl, Thiazolyl, Indolyl, Purinyl, Tetrahydrofuranyl, Dihydrofuranyl, Thiolanyl, Piperidinyl, Dihydropyranyl, Morpholinyl.
Bevorzugt sind dabei C₄-C₁₀-Heterocyclen mit ein bis zwei Heteroatomen aus der Gruppe O, S oder N
Kondensierte Ringsysteme, beispielsweise mit 6 bis 20 C Atomen, bestehen aus zwei oder mehreren kondensierten Ringe, wobei die Ringe aliphatisch oder aromatisch sein können und gegebenenfalls ein oder mehrere Heteroatome aus der Gruppe N, S oder O enthalten können. Beispiele sind etwa Indan, Tetralin, Chinolin, Chroman, Decalin, u.s.w.
Überbrückte Ringsysteme sind beispielsweise Bicyclo[2.2.1]heptan, Bicyclo[2.2.2]octan.

Die Reste können gegebenenfalls ein- oder mehrfach substituiert sein. Geeignete Substituenten sind dabei Alkyl, beispielsweise C₁-C₄-Alkyl, Phenyl, Halogen, Carbonsäurealkylester, beispielsweise C₁-C₆-Carbonsäureester mit 1 bis 4 C-Atomen im Esterteil, O-geschützte Hydroxy- und Hydroxyalkylgruppen.
Bevorzugte Substituenten sind C₁-C₂-Alkyl, Phenyl, Fluor, Chlor, C₁-C₃-Carbonsäure-C₁-C₂-alkylester, sowie durch eine Acetylgruppe geschützte Hydroxy- und Hydroxy-C₁-C₄-alkylgruppen.

Besonders bevorzugt bedeutet R1 einen gegebenenfalls ein- oder zweifach durch Fluor, Chlor, C₁-C₃-Carbonsäure-C₁-C₂-alkylester oder durch eine Acetylgruppe geschützte Hydroxy- oder Hydroxy-C₁-C₄-alkylgruppe substituierten Phenyl- oder Naphtytrest oder C₂-C₆-Alkylrest oder ein kondensiertes Ringsystem mit 6-10 C-Atomen. R2, R3 und R4 bedeuten R5 bedeutet besonders bevorzugt H oder einen C₁-C₆-Alkylrest.

R6 kann in der Formel (I) H oder eine N-Schutzgruppe bedeuten.
Als N-Schutzgruppen eignen sich alle gängigen N-Schutzgruppen, wie etwa Acetyl, Formyl, Chloroacetyl, Trichloracetyl, Phenylacetyl, Picolinoyl, Benzoyl, Carbamate wie z.B. Methyl, Ethyl, 9-Fluoroenylmethyl, 2,2,2,-Trichloroethyl, oder sonstige Schutzgruppen für Amine, wie z.B. in Theodora W. Greene, Peter G.M. Wuts Protective Groups In Organic Snthesis; Third Edition, Wiley Intersience beschrieben.

Die Allylverbindungen der Formel (I) werden erfindungsgemäß in zwei Schritten, durch Ozonolyse und anschließender oxidativer oder reduktiver Aufarbeitung zu den entsprechenden Aminoverbindungen der Formel (II) umgesetzt.

In der Formel (II) sind die Reste R1, R2, R3, R4 und R6 wie oben definiert.
Der Rest A bedeutet entweder eine Carboxylgruppe, sodass die Verbindung der Formel (II) eine beta-Aminosäure darstellt, oder eine Gruppe -C(OH)R5, in der R5 wie oben definiert ist, sodass die Verbindung der Formel (II) einen gamma-Aminoalkohol darstellt, oder eine Gruppe -C(O)R5, in der R5 wie oben definiert ist, sodass die Verbindung der Formel (II) ein beta-Aminoaldehyd oder -keton darstellt.

Im ersten Schritt erfolgt die Ozonolyse in einem Lösungsmittel
Als Lösungsmittel eignen sich beispielsweise C₁-C₆-Carbonsäuren, WasserlSchwefelsäuregemisch, C₁-C₄-Alkohol, Ethylacetat oder Butylacetat oder Gemischen davon.
Die Reaktionstemperatur wird in Abhängigkeit vom gewählten Lösungsmittel eingestellt und liegt bevorzugt bei -40 bis +30°C.

Sind beta-Aminosäuren der Formel (II) mit A gleich -COOH die gewünschten Endprodukte, so erfolgt die Ozonolyse der Verbindung der Formel (I), in der R5 H bedeutet, bevorzugt in einem Lösungsmittel aus der Gruppe der C₁-C₆-Carbonsäure oder in einem Wasser/Schwefelsäuregemisch.
Dazu wird die Verbindung der Formel (I) zuerst in einer C₁-C₆-Carbonsäure oder in einem Wasser/Schwefelsäuregemisch im Verhältnis 10:1 bis 50:1 aufgenommen und die so erhaltene Reaktionslösung auf eine Temperatur von 0 bis 30°C thermostatisiert.
Als Carbonsäuren werden dabei bevorzugt Essigsäure oder Propionsäure eingesetzt.
Anschließend erfolgt die Umsetzung mit Ozon, wobei Ozon in einer Menge von 1 bis 2 Äquivalenten in Form eines Ozon/Sauerstoffstromes zugeführt wird.

Sind die gamma-Aminoalkohole der Formel (II) mit A gleich C(OH)R5 oder beta-Aminoaldehyde oder -ketone der Formel (II) mit A gleich C(O)R5 die gewünschten Endprodukte so erfolgt die Ozonolyse der Verbindung der Formel (I) bevorzugt in einem C₁-C₆-Alkohol oder in Butylacetat oder Ethylacetat oder Gemischen davon aufgenommen und die so erhaltene Reaktionslösung auf eine Temperatur von -40 bis 0°C, bevorzugt auf -30 bis -10°C thermostatisiert.
Als Alkohol werden dabei bevorzugt Methanol oder Butanol eingesetzt.
Anschließend erfolgt die Umsetzung mit Ozon, wobei Ozon in einer Menge von 1 bis 2 Äquivalenten in Form eines Ozon/Sauerstoffstromes zugeführt wird.

Im zweiten Schritt erfolgt die Aufarbeitung der in Schritt eins erhaltenen Reaktionslösung.
Diese kann entweder durch Zersetzung der peroxidhaltigen Lösung mittels eines Oxidationsmittels oder durch reduktive Aufarbeitung erfolgen.

Ist die beta-Aminosäure das gewünschte Endprodukt, so wird nach beendeter Ozonolyse das peroxidhaltige Reaktionsgemisch bevorzugt auf 25°C bis zur Siedetemperatur des Lösungsmittels, bevorzugt auf 50 bis 70°C erwärmt und mit 1 bis 10 Äquivalenten, bevorzugt 4 bis 8 Äquivalenten, eines Oxidationsmittels versetzt.
Als Oxidaitonsmittel kommen übliche Oxidationsmittel wie etwa H₂O₂, tert-Butylhydroperoxid oder Sauerstoff in Frage. Bevorzugt wird H₂O₂ in Form einer 30 bis 70%igen Lösung eingesetzt.
Nach beendeter Peroxidzerstörung wird das Lösungsmittel/Wassergemisch abdestilliert und die gewünschte beta-Aminosäure gegebenenfalls durch Umkristallisieren oder Säulenchromatographie gereinigt. Im Falle eines SchwefelsäureNVasser-Gemisches wird nach beendeter Reaktion mit Lauge (z.B. NaOH) der pH-Wert so eingestellt, dass der isoelektrische Punkt der jeweiligen Aminosäure erreicht wird.
Dabei fällt die Aminosäure aus und wird abfiltriert mit Wasser gewaschen und getrocknet.

Die gewünschten beta-Aminosäuren werden dabei in Ausbeuten bis zu 99% d.Th. erhalten. Der Enantiomerenüberschuss der so erhaltenen beta-Aminosäuren entspricht dem der eingesetzten Verbindung der Formel (I).

Sind die gamma-Aminoalkohole die gewünschten Endprodukte, so erfolgt nach vollendeter Ozonolyse eine reduktive Aufarbeitung der erhaltenen Reaktionslösung in Gegenwart eines Reduktionsmittels.
Die reduktive Aufarbeitung wird dabei bevorzugt mit einem Reduktionsmittel aus der Gruppe NaBH₄ oder einem komplexen Hydrid durchgeführt.
Als Reduktionsmittel können somit beispielsweise NaBH₄, (R)-Alpine borane®, L-Selectride® oder andere komplexe Hydride mit oder ohne chiralen Liganden eingesetzt werden.
Dabei wird die Reaktionslösung in eine alkoholische Lösung, welche das Reduktionsmittel enthält zugegeben. Als Alkohol für die alkoholische Natriumborhydridiösung wird bevorzugt der Alkohol eingesetzt, der auch als bevorzugtes Lösungsmittel für die Ozonolyse verwendet wird.
Die Menge an Reduktionsmittel beträgt dabei 0,5 bis 4 mol pro mol Allylverbindung der Formel (I). Bevorzugt werden 0,5 bis 2 mol pro mol Allylverbindung der Formel (I) eingesetzt.
Anschließend wird die Reaktionslösung auf 10 bis 40°C, bevorzugt auf 20 bis 30°C erwärmt und mit 1-2 Äquivalenten Wasser, bezogen auf das Reduktionsmittel versetzt, um überschüssiges Reduktionsmittel zu zerstören.
Danach wird das Lösungsmittel abdestilliert und der Rückstand durch übliche Extraktionsmittel, wie etwa Dichlormethan, Ethylacetat, Butylacetat, MTBE ein- bis 5mal extrahiert. Die vereinigten organischen Phasen werden getrocknet, filtriert und abschließend vom Extraktionsmittel befreit. Gegebenenfalls können die beta-Aminoalkohole noch durch Umkristallisieren oder Säulenchromatographie gereinigt werden:

Die gewünschten gamma-Aminoalkohole werden dabei in Ausbeuten von bis zu 93% d.Th. erhalten. Der Enantiomerenüberschuss der so erhaltenen gamma-Aminoalkohole entspricht dem der eingesetzten Verbindung der Formel (I).

Sind die beta-Aminoaldehyde oder -ketone der Formel (IV) die gewünschten Endprodukte, so erfolgt nach vollendeter Ozonolyse ebenfalls eine reduktive Aufarbeitung der erhaltenen Reaktionslösung.

Die reduktive Aufarbeitung kann dabei beispielsweise mit Wasserstoff in Gegenwart eines Hydrierkatalysators erfolgen. Die katalytische Hydrierung kann dabei analog dem Stand der Technik, beispielsweise analog EP 147593; EP 99981 oder EP 1366008 durchgeführt werden.
Die Hydrierung erfolgt dabei in einem unter den Reaktionsbedingungen der Hydrierung inerten, organischen Verdünnungsmittel. Unter organischen Verdünnungsmitteln sind dabei neben den in der Ozonolyse verwendeten Lösungsmittel, übliche, bei der Hydrierung verwendete Verdünnungsmittel zu verstehen, wie beispielsweise aliphatische oder aromatische, gegebenenfalls chlorierte Kohlenwasserstoffe, wie Pentan, Hexan, Cyclohexan, Toluol, Xylole, Methylenchlorid, Dichlorethan, Chlorbenzole, Carbonsäureester wie Essigsäuremethyl-, -ethyl- oder - butylester, Ether und Ketone, sofern sie nicht zur Bildung sicherheitstechnisch bedenklicher Peroxide fähig sind, sowie Alkohole wie Methanol, Ethanol, iso-Propanol.
Als Katalysatoren eignen sich die für Hydrierungen üblicherweise verwendeten Edelmetallkatalysatoren, die in Form von Pulverkontakten mit Trägermaterialien oder ohne Trägermaterial eingesetzt werden können. Bevorzugt werden Palladium- oder Platinkatalysatoren verwendet, insbesondere Platinkatalysatoren ohne Trägermaterial. Bei Pulverkontakten eignen sich als Trägermaterial beispielsweise Kohle, Aluminium, Silikagel oder Kieselgur.
Die Menge an Wasserstoff, die bei der Hydrierung verwendet werden kann, reicht von einem Moläquivalent bis zu einem mehrfachen molaren Überschuss. Die Verwendung von überschüssigem Wasserstoff bringt an sich keine Vorteile und ist nur zweckmäßig, um eine ausreichende Versorgung des Hydriergemisches mit Wasserstoff sicherzustellen.
Die Hydrierung erfolgt beim erfindungsgemäßen Verfahren vorteilhafterweise unter praktisch drucklosen Bedingungen.
Unter praktisch drucklosen Bedingungen sollen hier Drucke von 1 bis etwa 8 bar verstanden werden, wie das in der Technik üblich ist, um das Eindringen von Luft in den Hydrierreaktor zu verhindern. Die reduktive Spaltung verläuft exotherm und wird bei 15 bis 40°C, bevorzugt bei Temperaturen im Bereich von 20 bis 40°C durchgeführt.
Zur Aufarbeitung des Reaktionsgemisches nach beendeter Hydrierung wird der Katalysator nach einer der bekannten Methoden, beispielsweise durch Filtrieren, Abdekantieren oder Zentrifugieren abgetrennt und das Lösungsmittel bevorzugt durch Abdestillieren zurückgewonnen.

Die reduktive Aufarbeitung kann aber auch durch Reduktion mittels Triphenylphosphin, Tributylphosphin, Thioharnstoff, organischen Sulfiden, wie z.B. Dimethylsulfid oder Bis-ethanolsulfid, oder mittels Zink in Essigsäure erfolgen.

Die gewünschten beta-Aminoaldehyde oder -ketone werden dabei in Ausbeuten von bis zu 90% d.Th. erhalten. Der Enantiomerenüberschuss der so erhaltenen beta- oder gamma-Aminoalkohole entspricht dem der eingesetzten Verbindung der Formel (I).
Sollte der erhaltene Aldehyd oder das Keton nicht ausreichend stabil sein, dann kann dieser in das Acetal oder Ketal oder in ein Bisulfitaddukt überführt werden. Das Schützen des Aldehydes oder Ketons kann auch in Lösung, welche nach der Ozonolyse und Hydrieung erhalten wird durchgeführt werden. Die Einführung der Schutzgruppe kann nach dem Stand der Literatur erfolgen wie z.B. beschrieben in: Theodora W. Greene and Peter G. M. Wuts; Protective Groups in Organic Synthesis, Third Edition, Wiley Interscience, 1999

### Beispiel 1-6:

### Arbeitsvorschrift für die Herstellung von chiralen Aminoalkoholen

0,04 mol ungeschütztes oder geschützes Allylamin wurden in 200 ml Methanol aufgenommen. Die Lösung wurde in ein Doppelmantelgefäß chargiert und auf -20°C abgekühlt. Nachdem ein konstanter Ozon/Sauerstoffstrom von 20 g/Nm³ eingestellt wurde, wurde mit der Ozonolyse begonnen. Nach beendeter Ozonolyse wurde die Reaktionslösung in eine eisgekühlte methanolische Natriumborhydridlösung (0,09 mol, 100 ml) innerhalb von 10 Minuten zugetropft. Danach wird die Reaktionslösung auf Raumtemperatur erwärmt und sodann mit 10 ml Wasser versetzt, um überschüssiges Natriumborhydrid zu zersetzen. Danach wurde das Lösungsmittel abdestilliert und der Rückstand mit Dichlormethan mehrmals extrahiert. Die vereinigten organischen Extrakte wurden über Natriumsulfat getrocknet, filtriert und danach das Lösungsmittel abdestilliert.
Gegebenenfalls wurde das erhaltene Produkt durch Umkristallisation oder Säulenchromatographie gereinigt.

### Beispiel 1:

**Ausgangsverbindung:** (*R*)-4-Amino-4-phenyl-1-buten
**Produkt**: (*R*)-3*-Amino-3-phenyl-1-propanol* wurde in 93 % Ausbeute und einen 99 % Enantiomerenüberschuss erhalten
Weise Kristalle ; mp 73 - 74 °C;
¹H-NMR (CDCl₃) 1.86 (m, 2H, -C*H*₂CH₂OH), 3.76 (t, 2H, -CH₂C*H*₂OH), 4.10 (t, 1H, - CH₂CH₂O*H*), 7.21 - 7.35 (m, 5H, Ar-*H*)

### Beispiel 2:

**Ausgangsverbindung**: (*R*)4-Amino-4-(4-pyridyl)-1-buten
**Produkt**: *(R)-3-Amino-3-(4-pyridyl)-1-propanol*
Ausbeute (nach Säulenchromatographie): 69 %
Enantiomerenüberschuss 40 %
Gelbes Öl; [α]_{D} = 33.24 (c = 1.02 g / ml, Chloroform);
¹H-NMR (CDCl₃) 1.95 (s, 3H, Ac-C*H*₃), 2.07 (s, 3H, Ac-C*H₃*), 2.18 (m, 2H, - C*H*₂CH₂OAc), 4.00 - 4.20 (m, 2H, -CH₂C*H*₂OAc), 5.13 (m, 1 H, -CH(NHAc)-), 7.26 - 8.50 (m, 4H, Pyr-*H*), 8.61 (br s, 1 H, -N*H*Ac) ppm;
¹³C-NMR (CDCl₃) 20.8 (Ac-CH₃), 22.9 (Ac-CH₃), 34.5 (-*C*H₂CH₂OAc), 48.5 (-CH(NHAc)-), 60.9 (-CH₂*C*H₂OAc), 123.9 (Pyr-C), 134.5 (Pyr-*C*), 138.0 (Pyr-*C*), 148.4 (Pyr-C), 148.5 (Pyr-*C*), 170.2 (Ac-CO), 170.9 (Ac-CO) ppm.

### Beispiel 3

**Ausgangsverbindung**: (*R*)-4-Amino-4-(4-fluorophenyl)-1-buten
**Produkt**: (*R*)-3*-Amino-3-(4-fluorophenyl)-1-propanol*
Ausbeute: 83 %
Enantiomerenüberschuss 87 %:
Weiße Kristalle; mp 141 - 142 °C; [α]_{D} = 22.61 (c = 1.99 g / ml, Chloroform);
¹H-NMR (CDCl₃) 1.84 (m, 2H, -*CH₂*CH₂OH), 3.17 (br s, 3H, -OH, -N*H₂*), 3.70 (m, 2H, CH₂C*H₂*OH), 4.10 (t, 1 H, -C*H*(NH₂)-), 6.91 - 7.29 (m, 4H, Ar-*H*) ppm;
¹³C-NMR (CDCl₃) 40.2 (-CH₂CH₂OH), 54.8 (-CHNH₂), 60.7 (-CH₂CH₂OH), 112.8 - 113.2 (Ar-C Pos. 4), 113.8 - 114.2 (Ar-C Pos. 6), 121.7 (Ar-C Pos. 3), 129.8 (Ar-C Pos. 2), 148.6 (Ar-C Pos. 1), 161.4 -164.7 (Ar-C Pos. 5) ppm.

### Beispiel 4:

**Ausgangsverbindung**: (R)-N-Acetyl-4-amino-4-phenyl-2-methyl-1-buten
**Produkt:** *(R,±)-N-Acetyl-4 Amino-4 phenyl-2-butanol*
Ausbeute: 84 %
Weiser Feststoff: mp 87 - 88 °C; [α]_{D} = 100.54 (c = 1.85 g / ml, Chloroform);
¹H-NMR (CDCl₃) 1.16 (d, 3H, -CH₂CH(C*H*₃)OH), 1.78 (m, 2H, -C*H*₂CH(CH₃)OH), 1.95 (s, 3H, Ac-C*H*₃), 3.76 (m, 1 H, -CH₂C*H*(CH₃)OH), 4.01 (br s, 1H, -O*H*), 4.95 - 5.18 (ddd, 1 H, -CH(NHAc)-), 7.07 (m, 1 H, -N*H*Ac), 7.20 - 7.32 (m, 5H, Ar-*H*) ppm;
¹³C-NMR (CDCl₃) □ 23.0 (-CH(*C*H₃)OH), 23.1 (Ac-CH₃), 45.2 (-CH₂CH(CH₃)OH), 51.0 (-CH(NHAc)-), 63.9 (-CH₂C*H*(CH₃)OH), 126.6 (Ar-C), 127.4 (Ar-C), 128.6 (Ar-*C*), 141.5 (Ar-C), 171.0 (Ac-CO) ppm.

### Beispiel 5:

**Ausgangsverbindung**: (R)-N-Acetyl-4-amino-4-phenyl-2-methyl-1-buten
**Produkt**: *(R,R)-N-Acetyl-4-Amino-4-phenyl-2-butanol*
Reduktion der Peroxidlösung wurde mit (R)-Alpine borane® , und mit L-Selectride® durchgeführt in Analogie zur Reduktion mit Natriumborhydrid.
Ausbeute nach Umkristallisation (aus Acetonitril) 76 %.
L-Selectride®: Diastereomerenverhältnis 1 : 3.
(*R*)-Alpine borane®: Diastereomerenverhältnis 1:2.
Farbloses Öl; [α]_{D} = 53.88 (c = 2.06 g / ml, Chloroform);
¹H-NMR (CDCl₃) 1.07 (m, 3H, -CH(CH₃)OH), 1.43 - 1.71 (m, 2H, -CH₂CH(CH₃)OH), 1.89 (s, 3H, Ac-C*H*₃), 3.51 (m, 1 H, -C*H*(CH₃)OH), 4.32 and 4.49 (2 s, 1 H, -OH), 4.94 (m, -CH(NHAc)-), 7.20 - 7.37 (m, 5H, Ar-*H*), 8.21 - 8.30 (2 d, 1 H, -N*H*Ac) ppm.

### Beispiel 6:

**Ausgangsverbindung**: (R)-N-Acetyl-4-Amino-5-methyl-1-hexen
**Produkt**: *(R)-N-Acetyl-3-Amino-4-methyl-1-pentanol*
Ausbeute: 93 %
Enantiomerenüberschuss: 89 %:
Weise Kristalle; mp 67 °C; [α]_{D} =11.29 (c = 1.86 g / ml, Chloroform);
¹H-NMR (CDCl₃) 0.94 (dd, 6H, -CH(C*H*₃)₂), 1.33 (m, 1 H, -C*H*(CH₃)₂), 1.71 - 1.87 (m, 2H, -C*H*₂CH₂OH), 2.03 (s, 3H, Ac-C*H*₃), 3.57 (m, 2H, -CH₂C*H₂*OH), 3.83 (m, 1H, - CH(NHAc)-), 3.96 (br s, 1H, -CH₂CH₂O*H*), 6.04 (d, 1 H, CH-N*H*Ac)-) ppm;
¹³C-NMR (CDCl₃) 18.9 (-CH(CH₃)₂), 19.7 (-CH(CH₃)₂), 23.4 (Ac-CH₃), 32.3 (-CH(CH₃)₂), 35.5 (-CH₂CH₂OH), 51.9 (-CH(NHAc)-), 59.2 (-CH₂CH₂OH), 172.0 (Ac-CO) ppm.

### Arbeitsvorschrift für die Herstellung von Aminoaldehydeniketonen

0,04 mol ungeschütztes oder geschützes Allylamin wurden in 200 ml Methanol aufgenommen. Die Lösung wurde in ein Doppelmantelgefäß chargiert und auf -20°C abgekühlt. Nachdem ein konstanter Ozon/Sauerstoffstrom von 20 g/Nm³ eingestellt wurde, wurde mit der Ozonolyse begonnen. Nach beendeter Ozonolyse wurde die Reaktionslösung mit Wasserstoff und einem Hydrierkatalysator z.B. Pd/C (5%) bei Normaldruck und 25°C hydrierert.
Danach wurde der Hydrierkatalysator durch Filtration abgetrennt und das Lösungsmittel abdestilliert.
Gegebenenfalls wurde das erhaltene Produkt durch Umkristallisation oder Säulenchromatographie gereinigt.

### Beispiel 7:

**Ausgangsverbindung**: (R)-N-Acetyl-4-amino-4-phenyl-2-methyl-1-buten
**Produkt**: *(R)-N-Acetyl-4-amino-4-phenyl-butan-2-one*
Ausbeute: 76 %
Enantiomerenüberschuss: 97 %:
Weiße Kristalle ; mp 77 - 78 °C; [α]_{D} = 50.94 (c = 2.12 g / ml, Chloroform);
¹H-NMR (CDCl₃) 1.95 (d, 3H, -CO-C*H₃*), 2.07 (s, 3H, Ac-C*H₃*), 2.77 - 3.07 (ddd, 2H, -C*H₂*COCH₃), 5.37 (dd, 1 H, -C*H*(NHAc)-), 7.04 (d, 1 H, -N*H*Ac), 7.20 - 7.32 (m, 5H, Ar-*H*) ppm;
¹³C-NMR (CDCl₃) 23.2 (Ac-CH₃), 30.5 (-CH₂COCH₃), 48.6 (-CH₂COCH₃), 49.6 (-CH(NHAc)-), 126.4 (Ar-C), 127.7 (Ar-C), 128.9 (Ar-C), 141.1 (Ar-C), 169.6 (Ac-CO), 207.3 (-COCH₃) ppm.

### Beispiel 8-11:

### Arbeitsvorschrift für die Herstellung von chiralen beta-Aminosäuren

0.02 mol Acetyl-geschütztes Allylamin wurde in 200 ml Essigsäure (technische Qualität) aufgenommen. Die Reaktionslösung wurde auf 18°C thermostatisiert Es wurde mit einem Ozon/Sauerstoffstrom mit einer Ozonkonzentration von 20 g / Nm³/h ozonisiert. Nachdem die Ozonolyse beendet war, wurde das Reaktionsgemisch auf 60°C erhitzt und mit 6 Äquivalenten Wasserstoffperoxid (50%, technische Qualität) versetzt. Nach 2-3 Stunden war die Reaktion beendet. Das Essigsäure/Wassergemisch wurde abdestilliert. Das Produkt wurde, falls notwendig, durch Umkristallisation aus Acetonitril oder Säulengromatograhpisch gereingt.

### Beispiel 8:

**Ausgangsverbindung:** (R)-4-N-Acetyl-amino-4-phenyl-1-buten
**Produkt:** *(R)-N-Acetyl-β-phenyl-β-alanine*
Ausbeute: 97 %
Enantiomerenüberschuss: 99 %,
Weiße Kristalle; mp 198 °C; [α]_{D} = 84.30 (c = 1.5 g / ml, Ethanol);
¹H-NMR (DMSO-d₆) 1.83 (s, 3H, Ac-*CH*₃), 2.68 (m, 2H,), 5.21 (m, 1 H,), 7.21 - 7.33 (m, 5H,), 8.38 (d, 1 H,), 12.26 (br s, 1 H,) ppm;
¹³C-NMR (DMSO-d₆) 23.0 (Ac-CH₃), 41.3 (-CH₂-COOH), 49.8 (-CH(NHAc)-), 126.8 (Ar-C), 127.3 (Ar-C), 128.6 (Ar-C), 143.0 (Ar-C), 168.6 (-COOH), 172.1 (Ac-*C*O) ppm.

### Beispiel 9:

**Ausgangsverbindung**: (*R*)-4-N-Acetyl-amino-4-(4-pyridyl)-1-buten
**Produkt**: *(R)-N-Acetyl-3-(4-pyridyl)-3-aminopropionic acid*
Ausbeute: 96 %
Enantiomerenüberschuss: 40 %
Hellgelbe Kristalle; mp 82 - 83 °C; [α]_{D} = 8.24 (c = 2.55 g /ml, Wasser);
¹H-NMR (CDCl₃) 1.89 (s, 3H, Ac-*CH*₃), 2.36 (t, 2H, -CH₂COOH), 5.00 - 5.15 (m, 1 H, -*CH*(NHAc)-), 7.30 - 8,55 (m, 4H, Pyr-*H*), 8.93 (m, 1 H, -N*H*Ac) ppm;
¹³C-NMR (CDCl₃) 24.3 (Ac-CH₃), 26.9 (-CH₂COOH), 50.3 (-CH(NHAc)-), 124.6 (Pyr-C), 135.5 (Pyr-C), 148.8 (Pyr-C), 149.8 (Pyr-C), 169.6 (Ac-CO), 176.4 (-COOH) ppm.

### Beispiel 10:

**Ausgangsverbindung**: (*R*)-4-N-Acetyl-amino-4-(4-fluorophenyl)-1-buten
**Produkt**: *(R)-N-Acetyl-3-(4-fluorophenyl)-3-aminopropionic acid*
Ausbeute: 99 %
Enantiomerenüberschuss: 89 %:
Weiße Kristalle; mp 33 °C; [α]_{D} = 69.60 (c = 2.27 g / ml, Methanol);
¹H-NMR (CDCl₃) 1.85 (s, 3H, Ac-CH₃), 2.67 (d, 2H, -C*H*₂COOH), 5.22 (m, 1H, - CH(NHAc)-), 7.01 - 7.39 (m, 5H, Ar-H), 8.40 (d, 1 H, -CH(NHAc)-) ppm;
¹³C-NMR (CDCl₃) 23.4 (Ac-CH₃), 41.6 (-CH₂COOH), 50.0 (-CH(NHAc)-), 114.0 (Ar-C), 114.4 (Ar-C), 123.4 (Ar-C), 130.9 (Ar-C), 146.5 (Ar-C), 161.4-164.6 (Ar-C), 169.3 (Ac-CO), 172.4 (-COOH) ppm.

### Beispiel 11:

**Ausgangsverbindung:** (R)-N-Acetyl-4-Amino-5-methyl-1-hexen
**Produkt**: *(R)-N-Acetyl-3-amino-4-methyl-pentanoic acid*
Ausbeute nach Säulenchromatographie: 58 %
Enantiomerenüberschuss 98 %
Gelbe Kristalle; mp 84 - 85 °C; [α]_{D} = - 29.03 (c = 2.17 g / ml, Chloroform);
¹H-NMR (CDCl₃) 0.93 (s, 6H, -CH(C*H₃*)₂), 2.00 (m, 3H, Ac-*CH₃*), 2.56 (s, 2H, -C*H₂-*COOH), 4.05 (s, 1 H, -CH(NHAc)-), 6.74 (br s, 1 H, -N*H*Ac) ppm;
¹³C-NMR (CDCl₃) 18.9 (-CH(CH₃)₂), 19.2 (-CH(*C*H₃)₂), 23.0 (Ac-CH₃), 40.2 (-*C*H₂-COOH), 51.9 (-CH(NHAc)-), 171.3 (Ac-CO), 175.7 (CH₂-COOH) ppm.

## Patentansprüche

1. Verfahren zur Herstellung von chiralen oder enantiomerenangereicherten beta-Aminosäuren, -aldehyden, -ketonen oder gamma-Aminoalkoholen, **dadurch gekennzeichnet, dass** ein Allylamin der Formel 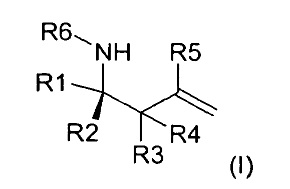 in der R1 einen Alkylrest, einen Cycloalkylrest, einen Arylrest, einen Heterocyclusrest oder ein kondensiertes oder überbrücktes Ringsystem bedeutet,
R2, R3, R4 und R5 unabhängig voneinander H oder einen Alkylrest, einen Cycloalkylrest, einen Arylrest, einen Heterocyclusrest oder ein kondensiertes oder überbrücktes Ringsystem bedeuten können,
oder die Reste R1, R2, R3 und R4 untereinander Ringsysteme bilden können, die gegebenenfalls ein oder mehrere Heteroatome enthalten können,
wobei die Reste R1, R2, R3, R4 und R5 gegebenenfalls ein- oder mehrfach durch Alkyl, Phenyl, Halogen, Carbonsäurealkylester, O-geschützte Hydroxy- und Hydroxyalkylgruppen substituiert sein können und
R6 H oder eine N-Schutzgruppe ist,
a) durch Ozonolyse in einem Lösungsmittel aus der Gruppe der C₁-C₆-Carbonsäure, Wasser/Schwefelsäuregemisch, C₁-C₄-Alkohol, Ethylacetat oder Butylacetat oder Gemischen davon bei einer Reaktionstemperatur, in Abhängigkeit vom gewählten Lösungsmittel, von -40°C bis +30°C und
b) anschließender Zersetzung der peroxidhaltigen Lösung mittels eines Oxidationsmittel oder reduktiver Aufarbeitung
in die entsprechende Aminoverbindung der Formel 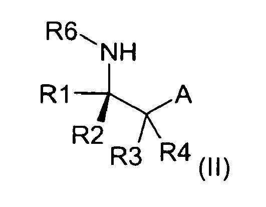 in der R1, R2, R3, R4 und R6 wie oben definiert sind,
und A in Abhängigkeit von der Aufarbeitung für einen Rest der Formel -COOH, -C(OH)R5 oder -C(O)R5 steht, wobei R5 wie oben definiert ist, überführt wird.

2. Verfahren nach Anspruch 1, **dadurch gekennzeichnet, dass** in der Formel (I) R1 einen C₁-C₂₀-Alkylrest, einen C₃-C₁₂-Cycloalkylrest, einen C₅-C₂₀-Arylrest, einen C₄-C₂₀-Heterocyclusrest oder ein kondensiertes oder überbrücktes Ringsystem mit 6 bis 20 C-Atomen bedeutet,
R2, R3, R4 und R5 unabhängig voneinander H oder einen C₁-C₂₀-Alkylrest, einen C₃-C₁₂-Cycloalkylrest, einen C₅-C₂₀-Arylrest, einen C₄-C₂₀-Heterocyclusrest oder ein kondensiertes oder überbrücktes Ringsystem mit 6 bis 20 C-Atomen bedeuten können,
oder die Reste R1, R2, R3 und R4 untereinander C₃-C₁₀-Ringsysteme bilden können, die gegebenenfalls ein oder mehrere Heteroatome enthalten können,
wobei die Reste R1, R2, R3, R4 und R5 gegebenenfalls ein- oder mehrfach durch C₁-C₄-Alkyl, Phenyl, Halogen, C₁-C₆-Carbonsäure-C₁-C₄-alkylester, O-geschützte Hydroxy- und Hydroxyalkylgruppen substituiert sein können und
R6 H oder eine N-Schutzgruppe ist.

3. Verfahren nach Anspruch 1, **dadurch gekennzeichnet, dass** die Ozonolyse für das Allyl der Formel (I), in der R1, R2, R3, R4 und R6 wie in Anspruch 1 definiert sind, und R5 gleich H ist , in einer C₁-C₆-Carbonsäure oder in einem Wasser/Schwefelsäuregemisch im Verhältnis 10:1 bis 50:1 als Lösungsmittel bei einer Temperatur von 0 bis 30°C durchgeführt wird.

4. Verfahren nach Anspruch 3, **dadurch gekennzeichnet, dass** als Lösungsmittel für die Ozonolyse Essigsäure oder Propionsäure eingesetzt wird.

5. Verfahren nach Anspruch 1 oder 2, **dadurch gekennzeichnet, dass**, falls gamma-Aminoalkohole der Formel (II) mit A gleich C(OH)R5 oder beta-Aminoaldehyde oder -ketone der Formel (II) mit A gleich C(O)R5 die gewünschten Endprodukte sind, die Ozonolyse in einem C₁-C₆-Alkohol oder in Butylacetat oder Ethylacetat oder in Gemischen davon als Lösungsmittel bei einer Temperatur von -40°C bis 0°C erfolgt.

6. Verfahren nach Anspruch 5, **dadurch gekennzeichnet**, als Lösungsmittel für die Ozonolyse Methanol oder Butanol eingesetzt wird.

7. Verfahren nach Anspruch 1, **dadurch gekennzeichnet, dass** bei der Zersetzung der peroxidhaltigen Lösung erhalten aus Schritt a) mittels eines Oxidationsmittels ein Oxidationsmittel aus der Gruppe H₂O₂, tert. Butylhydroperoxid oder Sauerstoff in einer Menge von 1 bis 10 Äquivalenten eingesetzt wird, und die Lösung auf 25°C bis zur Siedetemperatur des Lösungsmittels erwärmt wird.

8. Verfahren nach Anspruch 1, **dadurch gekennzeichnet, dass** falls beta-Aminosäuren der Formel (II) mit A gleich -COOH das gewünschte Endprodukt sind, die Zersetzung der peroxidhaltigen Lösung erhalten aus Schritt a) mittels eines Oxidationsmittels erfolgt.

9. Verfahren nach Anspruch 1, **dadurch gekennzeichnet, dass** falls beta-Aminosäuren der Formel (II) mit A gleich -COOH das gewünschte Endprodukt sind, die Ozonolyse gemäß Anspruch 5 und die Aufarbeitung der Ozonolyselösung gemäß Anspruch 9 erfolgt.

10. Verfahren nach Anspruch 8 oder 9, **dadurch gekennzeichnet, dass** nach beendeter Peroxidzerstörung das Lösungsmittel/Wassergemisch abdestilliert wird und die beta-Aminosäure gegebenenfalls durch Umkristallisieren oder Säulenchromatographie gereinigt wird.

11. Verfahren nach Anspruch 1, **dadurch gekennzeichnet**, dafür den Fall, dass Aminoalkohole der Formel (II) mit A gleich C(OH)R5 die gewünschten Endverbindungen sind, zur reduktiven Aufarbeitung gemäß Schritt b) ein Reduktionsmittel aus der Gruppe NaBH₄ oder der komplexen Hydride eingesetzt wird.

12. Verfahren nach Anspruch 11, **dadurch gekennzeichnet, dass** als Reduktionsmittel NaBH₄, (R)-Alpine borane®, L-Selectride® oder andere komplexe Hydride mit oder ohne chiralen Liganden eingesetzt werden.

13. Verfahren nach Anspruch 11, **dadurch gekennzeichnet, dass** 0,5 bis 4 mol an Reduktionsmittel pro mol Allylverbindung der Formel (I) eingesetzt werden.

14. Verfahren nach Anspruch 11, **dadurch gekennzeichnet, dass** nach beendeter reduktiver Aufarbeitung die Reaktionslösung auf 10 bis 40°C erwärmt wird und 1 bis 2 Äquivalente an Wasser bezogen auf das Reduktionsmittel zugesetzt werden um überschüssiges Reduktionsmittel zu zerstören.

15. Verfahren nach Anspruch 11, **dadurch gekennzeichnet, dass** die Isolierung des gamma-Aminoalkohols durch Extraktion erfolgt, wobei der beta-Aminoalkohol gegebenenfalls noch durch Umkristallisieren oder Säulenchromatographie gereinigt wird.

16. Verfahren nach Anspruch 1, **dadurch gekennzeichnet, dass** falls beta-Aminoaldehyde oder -ketone der Formel (II) mit A gleich C(O)R5 die gewünschten Endprodukte sind die reduktive Aufarbeitung gemäß Schritt b) mit Wasserstoff in Gegenwart eines Hydrierkatalysators oder durch Reduktion mit Triphenylphosphin, Tributylphosphin, Thioharnstoff, organischen Sulfiden oder durch Zink in Essigsäure erfolgt.

## Claims

1. Method for preparing chiral or enantiomer-enriched beta-amino acids, aldehydes, ketones or gamma-amino alcohols, **characterized in that** an allylamine of the formula 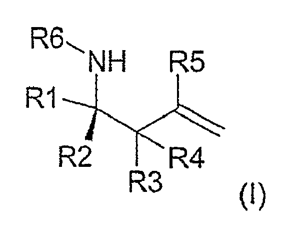 in which R1 is an alkyl radical, a cycloalkyl radical, an aryl radical, a heterocyclic radical or a fused or bridged ring system,
R2, R3, R4 and R5 may independently of one another be H or an alkyl radical, a cycloalkyl radical, an aryl radical, a heterocyclic radical or a fused or bridged ring system,
or the radicals R1, R2, R3 and R4 may form ring systems among themselves, which may optionally comprise one or more heteroatoms,
where the radicals R1, R2, R3, R4 and R5 may optionally be substituted one or more times by alkyl, phenyl, halogen, alkyl carboxylate, O-protected hydroxy and hydroxyalkyl groups, and
R6 is H or an N-protective group,
is converted
a) by ozonolysis in a solvent from the group of C₁-C₆-carboxylic acid, water/sulfuric acid mixture, C₁-C₄-alcohol, ethyl acetate or butyl acetate or mixtures thereof at a reaction temperature from -40°C to +30°C, depending on the chosen solvent, and
b) subsequent decomposition of the peroxide-containing solution using an oxidizing agent or reductive work-up
into the corresponding amino compound of the formula 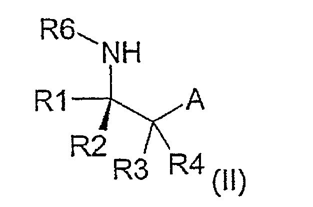
in which R1, R2, R3, R4 and R6 are as defined above,
and A is a radical of the formula -COOH, -C(OH)R5 or -C(O)R5, where R5 is as defined above, depending on the work-up.

2. Method according to Claim 1, **characterized in that** R1 in the formula (I) is a C₁-C₂₀-alkyl radical, a C₃-C₁₂-cycloalkyl radical, a C₅-C₂₀-aryl radical, a C₄-C₂₀-heterocyclic radical or a fused or bridged ring system having 6 to 20 C atoms,
R2, R3, R4 and R5 may independently of one another be H or a C₁-C₂₀-alkyl radical, a C₃-C₁₂-cycloalkyl radical, a C₅-C₂₀-aryl radical, a C₄-C₂₀-heterocyclic radical or a fused or bridged ring system having 6 to 20 C atoms,
or the radicals R1, R2, R3 and R4 may form C₃-C₁₀ ring systems among one another, which may optionally comprise one or more heteroatoms, where the radicals R1, R2, R3, R4 and R5 may optionally be substituted one or more times by C₁-C₄-alkyl, phenyl, halogen, C₁-C₄-alkyl C₁-C₆-carboxylate, O-protected hydroxy and hydroxyalkyl groups, and R6 is H or an N-protective group.

3. Method according to Claim 1, **characterized in that** the ozonolysis for the allyl of the formula (I) in which R1, R2, R3, R4 and R6 are as defined in claim 1, and R5 is H, is carried out in a C₁-C₆-carboxylic acid or in a water/sulfuric acid mixture in the ratio from 10:1 to 50:1 as solvent at a temperature of from 0 to 30°C.

4. Method according to Claim 3, **characterized in that** acetic acid or propionic acid is employed as solvent for the ozonolysis.

5. Method according to Claim 1 or 2, **characterized in that** if gamma-amino alcohols of the formula (II) with A equal to C(OH)R5 or beta-amino aldehydes or ketones of the formula (II) with A equal to C(O)R5 are the desired final products, the ozonolysis takes place in a C₁-C₆-alcohol or in butyl acetate or ethyl acetate or in mixtures thereof as solvent at a temperature from -40°C to 0°C.

6. Method according to Claim 5, **characterized in that** methanol or butanol is employed as solvent for the ozonolysis.

7. Method according to Claim 1, **characterized in that** an oxidizing agent from the group of H₂O₂, tert-butyl hydroperoxide and oxygen is employed in an amount of from 1 to 10 equivalents for the decomposition of the peroxide-containing solution resulting from step a) by means of an oxidizing agent, and the solution is heated to 25°C to the boiling point of the solvent.

8. Method according to Claim 1, **characterized in that** if beta-amino acids of the formula (II) with A equal to -COOH are the desired final product, the decomposition of the peroxide-containing solution resulting from step a) takes place by means of an oxidizing agent.

9. Method according to Claim 1, **characterized in that** if beta-amino acids of the formula (II) with A equal to -COOH are the desired final product, the ozonolysis takes place according to Claim 5 and the work-up of the ozonolysis solution takes place according to Claim 9.

10. Method according to Claim 8 or 9, **characterized in that** after the peroxide decomposition is complete, the solvent/water mixture is distilled off and the beta-amino acid is purified where appropriate by recrystallization or column chromatography.

11. Method according to Claim 1, **characterized in that** in the case where amino alcohols of the formula (II) with A equal to C(OH)R5 are the desired final compounds, a reducing agent from the group of NaBH₄ or of the complex hydrides is employed for the reductive work-up in step b).

12. Method according to Claim 11, **characterized in that** NaBH₄, (R)-Alpine borane®, L-Selectride® or other complex hydrides with or without chiral ligands are employed as reducing agent.

13. Method according to Claim 11, **characterized in that** from 0.5 to 4 mol of reducing agent are employed per mol of allyl compound of the formula (I).

14. Method according to Claim 11, **characterized in that** after the reductive work-up is complete, the reaction solution is warmed to 10 to 40°C, and 1 to 2 equivalents of water, based on the reducing agent, are added in order to decompose excess reducing agent.

15. Method according to Claim 11, **characterized in that** the gamma-amino alcohol is isolated by extraction, with the beta-amino alcohol also being purified where appropriate by recrystallization or column chromatography.

16. Method according to Claim 1, **characterized in that** if beta-amino aldehydes or ketones of the formula (II) with A equal to C(O)R5 are the desired final products, the reductive work-up in step b) takes place with hydrogen in the presence of a hydrogenation catalyst or by reduction with triphenylphosphine, tributylphosphine, thiourea, organic sulfides or by zinc in acetic acid.

## Revendications

1. Procédé pour la préparation de bêta-aminoacides, bêta-aminoaldéhydes, bêta-aminocétones ou de gamma-aminoalcools chiraux ou enrichis en énantiomères, **caractérisé en ce qu'**on transforme une allylamine de formule 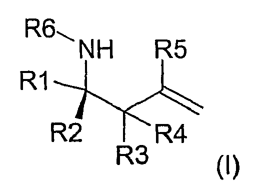 dans laquelle
R1 signifie un radical alkyle, un radical cycloalkyle, un radical aryle, un radical hétérocyclyle ou un système cyclique fusionné ou ponté,
R2, R3, R4 et R5 peuvent signifier, indépendamment l'un de l'autre, H ou un radical alkyle, un radical cycloalkyle, un radical aryle, un radical hétérocyclyle ou un système cyclique fusionné ou ponté,
ou les radicaux R1, R2, R3 et R4 peuvent former les uns avec les autres des systèmes cycliques, qui peuvent le cas échéant contenir un ou plusieurs hétéroatomes,où les radicaux R1, R2, R3, R4 et R5 peuvent le cas échéant être monosubstitués ou polysubstitués par alkyle, phényle, halogène, ester alkylique d'acide carboxylique, des groupes hydroxy ou hydroxyalkyle à O protégé et
R6 représente H ou un groupe de protection de N,
a) par ozonolyse dans un solvant du groupe formé par les acides C₁-C₆-carboxyliques, un mélange eau/acide sulfurique, un alcool en C₁-C₄, l'acétate d'éthyle ou l'acétate de butyle ou des mélanges de ceux-ci à une température de réaction, en fonction du solvant choisi, de -40°C à +30°C et
b) décomposition consécutive de la solution contenant un peroxyde au moyen d'un oxydant ou d'un traitement réducteur
en composé amino correspondant de formule 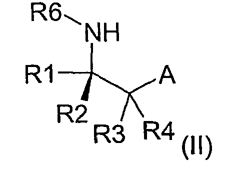 dans laquelle R1, R2, R3, R4 et R6 sont définis comme ci-dessus,
et A représente, en fonction du traitement, un radical de formule -COOH, -C(OH)R5 ou -C(O)R5, R5 étant défini comme ci-dessus.

2. Procédé selon la revendication 1, **caractérisé en ce que**, dans la formule (I)
R1 signifie un radical alkyle en C₁-C₂₀, un radical cycloalkyle en C₃-C₁₂, un radical aryle en C₅-C₂₀, un radical hétérocyclyle en C₄-C₂₀ ou un système cyclique fusionné ou ponté comprenant 6 à 20 atomes de carbone,
R2, R3, R4 et R5 peuvent signifier, indépendamment l'un de l'autre, H ou un radical alkyle en C₁-C₂₀, un radical cycloalkyle en C₃-C₁₂, un radical aryle en C₅-C₂₀, un radical hétérocyclyle en C₄-C₂₀ ou un système cyclique fusionné ou ponté comprenant 6 à 20 atomes de carbone,
ou les radicaux R1, R2, R3 et R4 peuvent former, les uns avec les autres, des systèmes cycliques en C₃-C₁₀, qui peuvent le cas échéant contenir un ou plusieurs hétéroatomes, où les radicaux R1, R2, R3, R4 et R5 peuvent le cas échéant être monosubstitués ou polysubstitués par alkyle en C₁-C₄, phényle, halogène, ester C₁-C₄-alkylique d'un acide carboxylique en C₁-C₆, des groupes hydroxy et hydroxyalkyle à 0 protégé et
R6 représente H ou un groupe de protection de N.

3. Procédé selon la revendication 1, **caractérisé en ce que** l'ozonolyse pour l'allyle de formule (I), dans laquelle R1, R2, R3, R4 et R6 sont définis comme dans la revendication 1 et R5 représente H, est réalisée dans un acide carboxylique en C₁-C₆ ou dans un mélange eau/acide sulfurique dans un rapport 10:1 à 50:1 comme solvant à une température de 0 à 30°C.

4. Procédé selon la revendication 3, **caractérisé en ce qu'**on utilise, comme solvant pour l'ozonolyse, de l'acide acétique ou de l'acide propionique.

5. Procédé selon la revendication 1 ou 2, **caractérisé en ce que**, dans le cas où des gamma-aminoalcools de formule (II) avec A représentant C(OH)R5 ou des bêta-aminoaldéhydes ou des bêta-aminocétones de formule (II) avec A représentant C(O)R5 seraient les produits finaux souhaités, l'ozonolyse est réalisée dans un alcool en C₁-C₆ ou dans de l'acétate de butyle ou de l'acétate d'éthyle ou dans des mélanges de ceux-ci comme solvants à une température de -40°C à 0°C.

6. Procédé selon la revendication 5, **caractérisé en ce qu'**on utilise, comme solvant pour l'ozonolyse, du méthanol ou du butanol.

7. Procédé selon la revendication 1, **caractérisé en ce qu'**on utilise, lors de la décomposition de la solution contenant un peroxyde, obtenue après l'étape a), au moyen d'un oxydant, un oxydant du groupe formé par H₂O₂, tert-butylhydroperoxyde ou oxygène, en une quantité de 1 à 10 équivalents, et la solution est réchauffée à 25°C jusqu'à la température d'ébullition du solvant.

8. Procédé selon la revendication 1, **caractérisé en ce que**, dans le cas où des bêta-aminoacides de formule (II) avec A représentant -COOH seraient le produit final souhaité, la décomposition de la solution contenant un peroxyde obtenue après l'étape a) est réalisée au moyen d'un oxydant.

9. Procédé selon la revendication 1, **caractérisé en ce que**, dans le cas où des bêta-aminoacides de formule (II) avec A représentant -COOH seraient le produit final souhaité, l'ozonolyse est réalisée selon la revendication 5 et le traitement de la solution d'ozonolyse selon la revendication 9.

10. Procédé selon la revendication 8 ou 9, **caractérisé en ce qu'**après la fin de la décomposition du peroxyde, le mélange solvant/eau est éliminé par distillation et le bêta-aminoacide est le cas échéant purifié par recristallisation ou chromatographie sur colonne.

11. Procédé selon la revendication 1, **caractérisé en ce que**, pour le cas où des aminoalcools de formule (II) avec A représentant C(OH)R5 seraient les composés finaux souhaités, on utilise, pour le traitement réducteur selon l'étape b), un réducteur du groupe formé par NaBH₄ ou les hydrures complexes.

12. Procédé selon la revendication 11, **caractérisé en ce qu'**on utilise comme réducteur du NaBH₄, du (R)-Alpine borane®, du L-Selectride® ou d'autres hydrures complexes avec ou sans ligands chiraux.

13. Procédé selon la revendication 11, **caractérisé en ce qu'**on utilise 0,5 à 4 moles de réducteur par mole de composé allyle de formule (I).

14. Procédé selon la revendication 11, **caractérisé en ce qu'**à la fin du traitement réducteur, la solution réactionnelle est réchauffée à 10 jusqu'à 40°C et 1 à 2 équivalents d'eau, par rapport au réducteur, sont ajoutés pour décomposer le réducteur en excès.

15. Procédé selon la revendication 11, **caractérisé en ce que** l'isolement du gamma-aminoalcool est réalisé par extraction, le bêta-aminoalcool étant le cas échéant encore purifié par recristallisation ou chromatographie sur colonne.

16. Procédé selon la revendication 1, **caractérisé en ce que**, dans le cas où des bêta-aminoaldéhydes ou des bêta-aminocétones de formule (II) avec A représentant C(O)R5 seraient les produits finaux souhaités, le traitement réducteur selon l'étape b) est réalisé avec de l'hydrogène en présence d'un catalyseur d'hydrogénation ou par réduction avec de la triphénylphosphine, de la tributylphosphine, de la thio-urée, des sulfures organiques ou par du zinc dans de l'acide acétique.
